# EUROPEAN PATENT APPLICATION

(11) **EP 3 949 928 A1**
(43) Date of publication of application: **09.02.2022**
(21) Application number: 20779706.9
(22) Date of filing: 12.03.2020
(51) Int. Cl.: A61H 3/00

(54) **JOINT MECHANISM, KNEE JOINT ASSIST DEVICE, JOINT MEMBER, AND CLUTCH UNIT**

(30) Priority: 25.03.2019 JP 2019057479
(71) Applicant: Fujikura Kasei Co., Ltd., Tokyo 174-0046 (JP)
(72) Inventor: ANZAI Hidenobu, Kuki-shi Saitama 340-0203 (JP); NAGATSUMA Akemi, Kuki-shi Saitama 340-0203 (JP); MITSUI Kazuyuki, Higashiyamato-shi Tokyo 207-0023 (JP)
(74) Representative: Oppermann, Frank
(86) International application number: PCT/JP2020/010862
(87) International publication number: WO 2020/195906

(57) **Abstract**

Provided is a joint mechanism capable of locking at the time of completely extending or in the middle of the extending while suppressing complication and weigh increase of the device. The joint mechanism has a leg frame part 15a and a thigh frame part 13a pivotally connected to each other, a one-way clutch 37 connected to the leg frame part 15a and the thigh frame part 13a so as to lock turning between the leg frame part 15a and the thigh frame part 13a and allow the turning in one direction only, and a connecting/disconnecting part 21 to connect and disconnect the connecting state between the one-way clutch 37 and the thigh frame part 13a, wherein the connecting/disconnecting part 21 disconnects between the one-way clutch 37 and the leg frame part 15a to unlock the turning between the leg frame part 15a and the thigh frame part 13a to allow the turning of the leg frame part 15a and the thigh frame part 13a in both directions.

## Description

### FIELD OF THE INVENTION

The present invention relates to a joint mechanism, a knee joint assist device, a joint member, and a clutch unit capable of being locked and unlocked.

### BACKGROUND OF THE INVENTION

As a device or the like for walking assistance or rehabilitation for an aided person, a walking motion assistance device being a knee joint assist device is used, for example. As the walking motion assistance device, one being provided with an actuator such as electric motor is proposed as Patent document 1.

The walking motion assistance device pivotally connects a thigh side orthosis to be attached to a thigh of an aided person and a leg side orthosis to be attached to a leg using a joint mechanism and is configured to apply torque to the joint mechanism using an actuator such as electric motor.

With this, when an aided person walks, the torque is applied to the joint mechanism to be locked so as not to bend between the thigh side orthosis and the leg side orthosis during, for example, a term from putting a heel of a lower limb on a floor until leaving the heel from the floor, to prevent a knee bending of the aided person. Further, after leaving the heel according to the walking, reverse torque is applied to the joint mechanism to release the locking and assist a motion to swing forward the lower limb until the heel is subsequently put on the floor.

In this way, the walking motion assistance device of Patent document 1 assists the walking motion of the aided person to allow the walking motion to be smoothly performed.

In order to make the gait of the aided person natural, however, it is necessary to conduct the locking when the thigh and the leg (of the joint mechanism) are completely extended or in the middle of the extending according to the gait of the aided person. Accordingly, the walking motion assistance device of Patent document 1 is provided with various sensors and has a problem of causing complication and weight increase of the device.

This problem is also caused in assistance devices for other joints such as ankle, elbow, and shoulder and in joints for robots to be locked using an actuator such as electric motor when a joint mechanism is completely extended or in the middle of the extending. Further, some joints need to be locked at the time of completely bending or in the middle of the bending and a similar problem is caused in such cases.

### PRIOR ART DOCUMENT

### PATENT DOCUMENT

PATENT DOCUMENT 1: JP 2018-050881 A

### SUMMARY OF THE INVENTION

### PROBLEMS TO BE SOLVED BY THE INVENTION

A problem to be solved is to cause complication and weight increase of a device in order to lock a joint mechanism at the time of completely extending or in the middle of the extending or at the time of completely bending or in the middle of the bending.

### MEANS FOR SOLVING THE PROBLEM

The present invention mainly characterizes a joint mechanism by a first turnable member and a second turnable member pivotally connected to each other, a one-way clutch connected to the first turnable member and the second turnable member so as to lock turning between the first turnable member and the second turnable member and allow the turning in one direction only, and a connecting/disconnecting part to connect and disconnect a connecting state between the one-way clutch and the second turnable member, wherein the connecting/disconnecting part disconnects between the one-way clutch and the second turnable member to release the locking of the turning to allow the first turnable member and the second turnable member to turn in both directions.

### EFFECT OF THE INVENTION

The present invention connects between the one-way clutch and the second turnable member using the connecting/disconnecting part to lock the turning between the first turnable member and the second turnable member so as to allow the turning in an extending direction or a bending direction only.

As this result, if the locking is performed in the process of the extending or the bending between the first turnable member and the second turnable member, operation in the extending direction or the bending direction is kept on performing between the first turnable member and the second turnable member and it is possible to perform the locking in the state of completely extending or in the middle of the extending or in the state of completely bending or in the middle of the bending.

Accordingly, the joint mechanism is lockable in the state of completely extending or in the middle of the extending or in the state of completely bending or in the middle of the bending without complicated control with the simple structure while suppressing complication and weight increase of the device.

### BRIEF DESCRIPTION OF THE DRAWINGS

[FIG. 1] It is a perspective view illustrating a long lower limb brace as a knee joint assist device to which a joint mechanism is applied (Embodiment 1).
[FIG. 2] It is a perspective view illustrating the joint mechanism used in the long lower limb brace of FIG. 1 (Embodiment 1).
[FIG. 3] It is a side view illustrating an enlarged essential part of the joint mechanism of FIG. 2 (Embodiment 1).
[FIG. 4] It is a bottom view illustrating the enlarged essential part of the joint mechanism of FIG. 2 (Embodiment 1).
[FIG. 5] It is a perspective view illustrating a joint member used in the joint mechanism of FIG. 2 (Embodiment 1).
[FIG. 6] It is an exploded perspective view illustrating the joint member of FIG. 5 (Embodiment 1).
[FIG. 7] It is a plan view illustrating an enlarged essential part of the joint member of FIG. 5 (Embodiment 1).
[FIG. 8] It is a perspective sectional view of the joint member of FIG. 5 taken along a line VIII-VIII (Embodiment 1).
[FIG. 9] It is a sectional view of the joint member of FIG. 5 taken along the line VIII-VIII (Embodiment 1).
[FIG. 10] It is a perspective view of a connecting/disconnecting part used in the joint mechanism of FIG. 2 when viewed from a bottom side (Embodiment 1).
[FIG. 11] It is a block diagram illustrating the connecting/disconnecting part of FIG. 10 (Embodiment 1).
[FIG. 12] It is a schematic view illustrating a relation between motion of the joint member and locking (Embodiment 1).
[FIG. 13] It is a sectional view illustrating a joint mechanism (Embodiment 2).
[FIG. 14] It is a block diagram illustrating a connecting/disconnecting part (Embodiment 3).

### EMBODIMENT FOR CARRYING OUT THE INVENTION

The present invention accomplishes the object, capable of performing locking a joint mechanism in a state of completely extending or in the middle of the extending or in a state of completely bending or in the middle of the bending while suppressing complication and weight increase of the device, by the joint mechanism connecting a one-way clutch between a first turnable member and a second turnable member that are pivotally connected to each other and connecting and disconnecting between the one-way clutch and the second turnable member using a connecting/disconnecting part.

Namely, the joint mechanism is provided with the first turnable member and the second turnable member pivotally connected to each other, the one-way clutch connected to the first turnable member and the second turnable member so as to lock turning between the first turnable member and the second turnable member and allow the turning in one direction only, and the connecting/disconnecting part to connect and disconnect a connecting state between the one-way clutch and the second turnable member, wherein the connecting/disconnecting part disconnects between the one-way clutch and the second turnable member to release the locking of the turning to allow the first turnable member and the second turnable member to turn in both directions.

The joint mechanism may further comprise a connecting part provided to the one-way clutch to connect the second turnable member, and a displacement part provided to the second turnable member and configured to displace relatively to the connecting part according to the turning between the first turnable member and the second turnable member during the disconnecting of the one-way clutch and the second turnable member, wherein the connecting/disconnecting part connects between the connecting part and the displacement part to disable for the displacing, thereby to connect the second turnable member to the one-way clutch.

The connecting part and the displacement part may be arranged to overlap each other in a turning axis direction, be relatively rotatable around a turning axis, and have communicating holes communicating in the turning axis direction, and the connecting/disconnecting part may have a pin arranged in the communicating holes so as to be movable in an axis direction, and perform the connecting and the disconnecting according to the moving of the pin in the axis direction.

The connecting part and the displacement part may be arranged apart from each other in an intersecting direction intersecting the turning axis and are relatively turnable around the turning axis, the connecting/disconnecting part may comprise a body portion having a first engagement portion engaging with the displacement part, a rotational part supported with the body portion rotatably around the turning axis and having a second engagement portion engaging with the connecting part, and a brake part configured to brake the rotation of the rotational part and release the braking to conduct the connecting and disconnecting.

The connecting/disconnecting part may be detachably attached to the one-way clutch.

A knee joint assist device using the joint mechanism may be provided with a thigh orthosis provided to one of the first turnable member and the second turnable member of the joint mechanism to be attached to a thigh of an aided person, and a leg orthosis provided to the other of the first turnable member and the second turnable member of the joint mechanism to be attached to a leg of the aided person, wherein the joint mechanism locks the turning between the first turnable member and the second turnable member to allow the thigh orthosis and the leg orthosis to turn in an extending direction only and releases the locking of the turning to allow the thigh orthosis and the leg orthosis to turn in both directions of the extending direction and a bending direction.

The knee joint assist device may further comprise a sensor that detects a front/rear position of the thigh of the aided person, wherein the joint mechanism is capable of electrically switching the locking of the turning and the releasing the locking, the locking of the turning is performed when the thigh of the aided person is positioned ahead of a center of gravity of a body of the aided person and the locking of the turning is released when the thigh of the aided person is positioned behind the center of gravity of the body of the aided person based on the detecting of the sensor.

Ajoint member composing the joint mechanism may be provided with the first turnable member and the second turnable member pivotally connected to each other, the one-way clutch connected to the first turnable member and the second turnable member to lock the turning between the first turnable member and the second turnable member and allow the turning in one direction only, and the attachment part for attaching the connecting/disconnecting part to connect and disconnect the connecting state between the one-way clutch and the second turnable member.

A clutch unit used for the joint mechanism may be provided with a first attachment member and a second attachment member engaging with the first turnable member and the second turnable member in the turning direction, a fixing member detachably fixing the first attachment member or the second attachment member to the first turnable member or the second turnable member, the one-way clutch supported with one of the first attachment member and the second attachment member to lock the turning between the first turnable member and the second turnable member and allow the turning in one direction only, a relatively rotatable part provided to the other of the first attachment member and the second attachment member and relatively rotatably supported with an outer periphery of the one-way clutch, and the attachment part for attaching the connecting/disconnecting part to connect and disconnect the connecting state between the one-way clutch and the second turnable member.

### EMBODIMENT 1

### [Long lower limb brace]

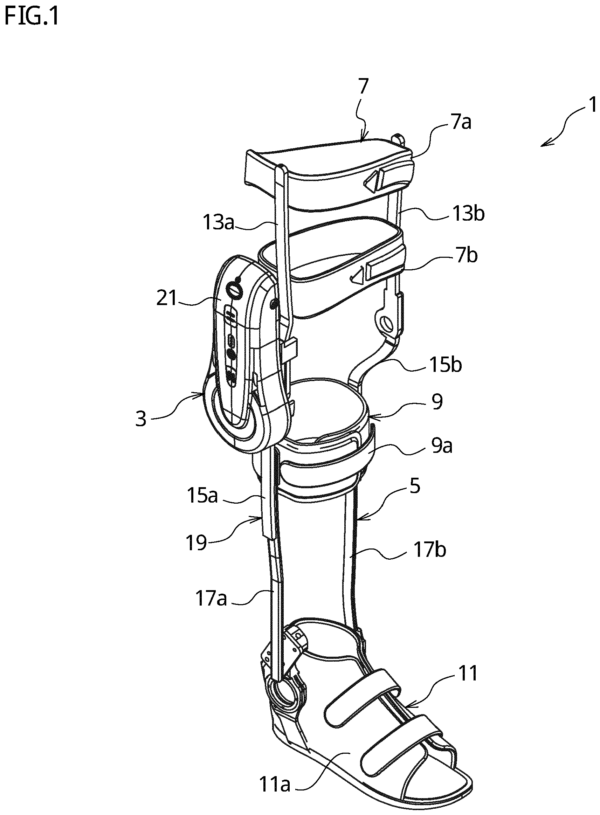

FIG. 1 is a perspective view illustrating a long lower limb brace as a knee joint assist device to which a joint mechanism according to the embodiment 1 of the present invention is applied.

As illustrated in FIG. 1, the long lower limb brace 1 is a device for walking assistance or rehabilitation and is used so as to be attached to a lower limb of an aided person. In addition, the long lower limb brace 1 of FIG. 1 is for a right lower limb. A long lower limb brace for a left lower limb would be configured symmetrically with the long lower limb brace 1.

Hereinafter, upper and lower, right and left, and back and forth means upper and lower, right and left, and back and forth in the state that the long lower limb brace 1 is attached to the aided person, respectively. Further, an axial direction is a turning axis direction along an axis of a turning shaft 25 of a joint mechanism 3 explained later. A turning direction is a direction along which thigh frame parts 13a, 13b and leg frame parts 15a, 15b of the joint mechanism 3 turn.

The long lower limb brace 1 of the present embodiment is provided with a frame 5 having the joint mechanism 3, a thigh orthosis 7, a leg orthosis 9, and a foot orthosis 11.

The frame 5 is configured by pivotally connecting the thigh frame parts 13a, 13b positioned on the thigh side and the leg frame parts 15a, 15b positioned on the leg side to each other. Although in the frame 5 of the present embodiment extended frame parts 17a, 17b being extended downward are connected to the leg frame parts 15a, 15b, the extended frame parts 17a, 17b may be omitted.

The thigh frame part 13a and the leg frame part 15a on one side as the respective second turnable member and first turnable member of the present embodiment compose a joint member 19, and are provided with a one-way clutch 37 as explained later. The joint member 19 forms the joint mechanism 3 upon attachment of a connecting/disconnecting part 21. In addition, the joint member 19 of the present embodiment does not include the extended frame part 17a, but may include the extended frame part 17a. Further, in the joint member 19, the thigh frame part 13a may be the first turnable member and the leg frame part 15a may be the second turnable member.

It should be noted that the frame 5 may be formed by the thigh frame part 13a, the leg frame part 15a, and the extended frame part 17a which form the joint mechanism 3 with absence of the thigh frame part 13b, the leg frame part 15b, and the extended frame part 17b.

The thigh orthosis 7 has belt-shaped fitting members 7a, 7b attached between the thigh frame parts 13a, 13b of the frame 5. In addition, the fitting members 7a, 7b are not limited to the belt shape. The thigh orthosis 7 is detachably attached to a thigh of an aided person using the fitting members 7a, 7b.

The leg orthosis 9 has a belt-shaped fitting member 9a attached between the leg frame parts 15a, 15b of the frame 5, and is detachably attached to a leg of an aided person using the fitting member 9a. The fitting member 9a of the leg orthosis 9 is not limited to the belt shape either. The leg orthosis 9 is configured to be turnable relatively to the thigh orthosis 7 through the frame 5.

The foot orthosis 11 is pivotably attached to lower ends of the extended frame parts 17a, 17b of the frame 5, and has a shoe-shaped fitting member 11a. With this fitting member 11a, the foot orthosis 11 is detachably attached to a foot of an aided person.

In addition, the foot orthosis 11 may be omitted. Further, the fitting member 11a of the foot orthosis 11 may not be the shoe shape, but a belt shape or the like.

The long lower limb brace 1, when an aided person puts on it and walks, locks the turning between the thigh orthosis 7 and the leg orthosis 9 and release the locking according to the connecting/disconnecting part 21 of the joint mechanism 3, thereby to make the aided person a natural gait. The details will be explained later.

### [Joint mechanism]

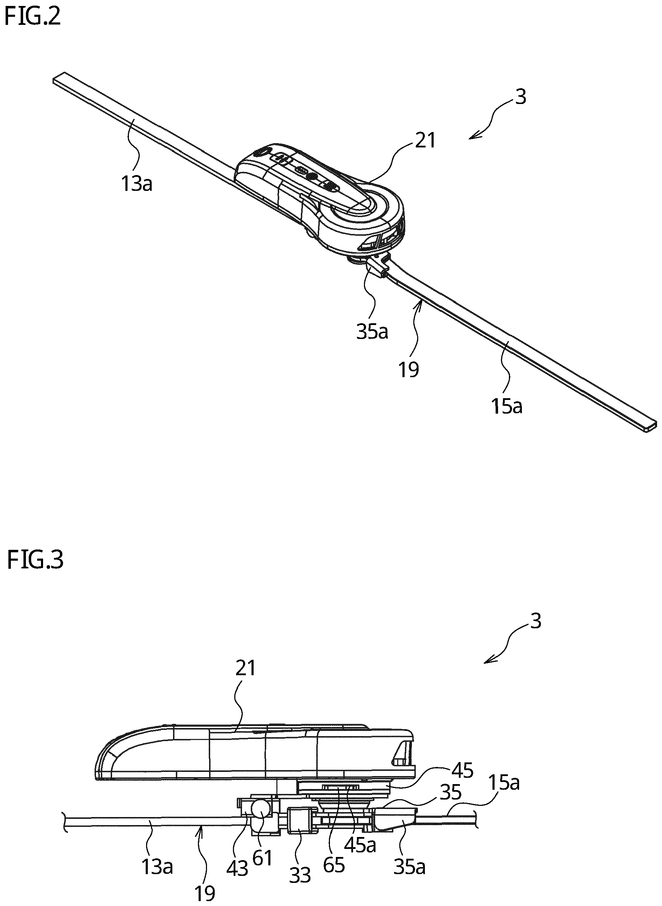

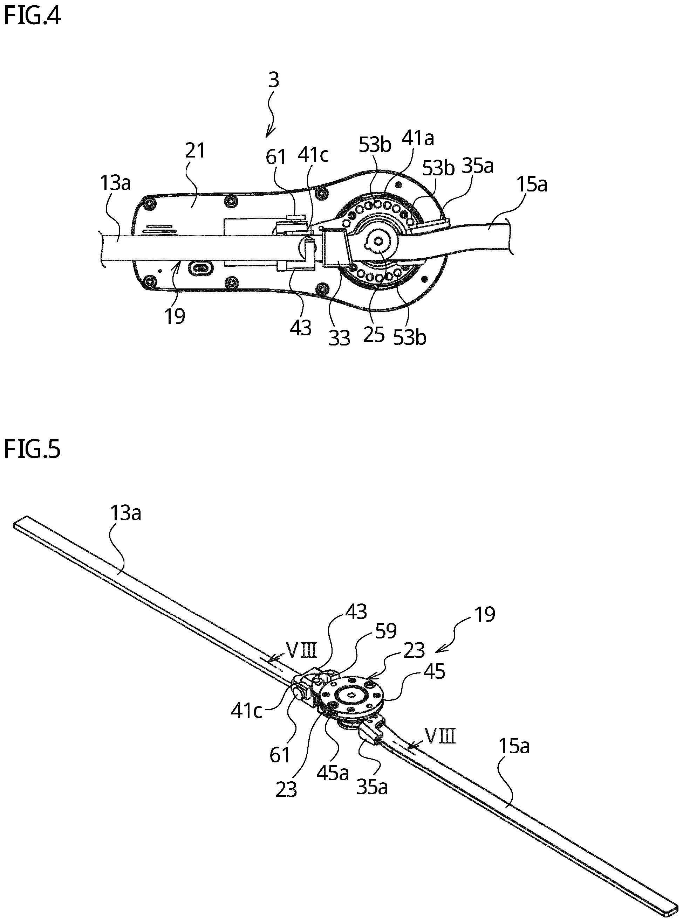

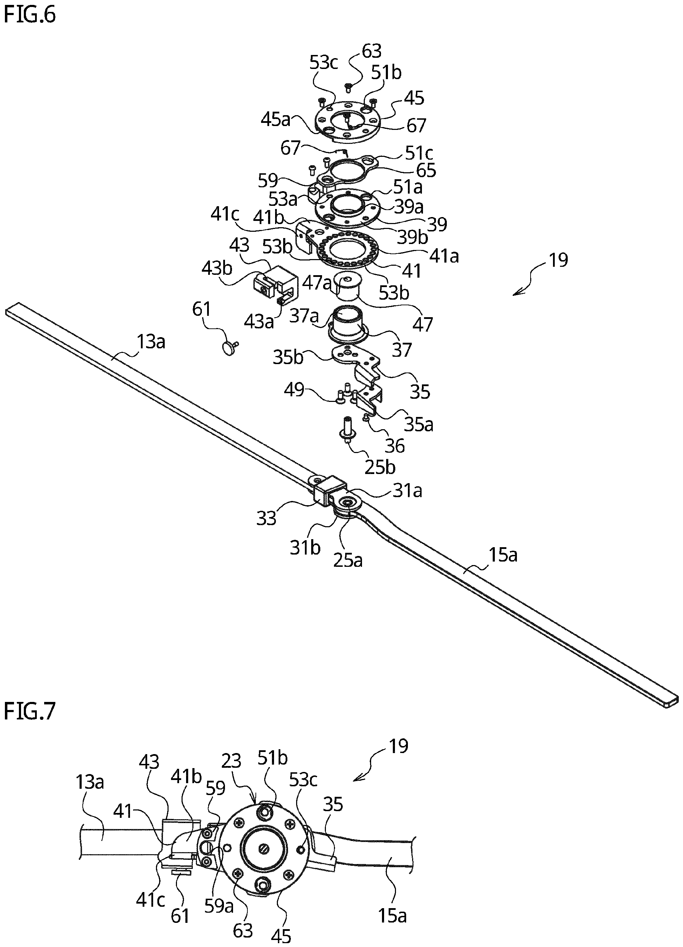

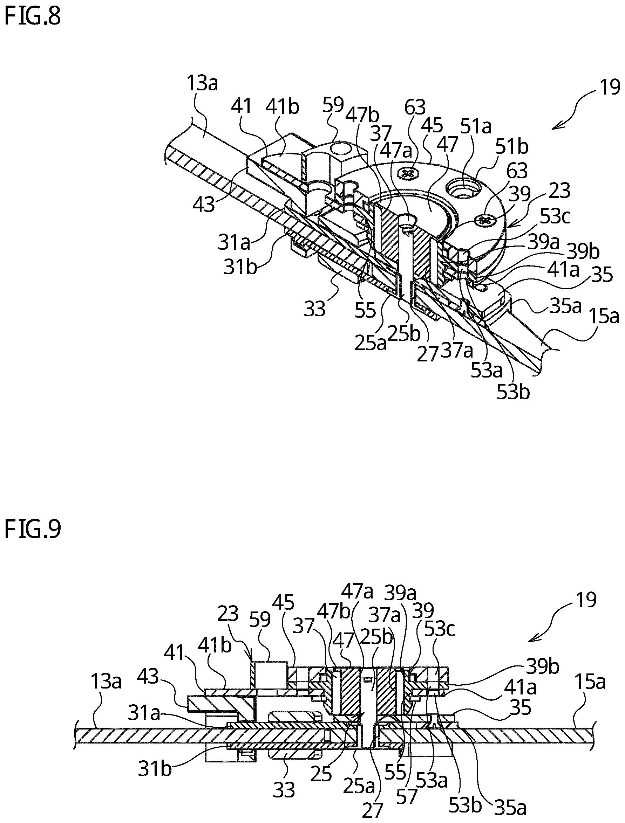

FIG. 2 is a perspective view illustrating the joint mechanism 3 used in the long lower limb brace 1 of FIG. 1, FIG. 3 is a side view illustrating an enlarged essential part of the joint mechanism 3 of FIG. 2, FIG. 4 is a bottom view of the same, FIG. 5 is a perspective view illustrating the joint member 19 used in the joint mechanism 3 of FIG. 2, FIG. 6 is an exploded perspective view illustrating the joint member 19 of FIG. 5, FIG. 7 is a plan view illustrating an enlarged essential part of the joint member 19 of FIG. 5, FIG. 8 is a perspective sectional view of the joint member 19 of FIG. 5 taken along a line VIII-VIII, and FIG. 9 is a sectional view of the same.

The joint mechanism 3 is provided with the joint member 19, and the connecting/disconnecting part 21 attached to the joint member 19. Although the joint mechanism 3 of the present embodiment is used as a part of the long lower limb brace 1, it may be used to, for example, assist devices for other joints such as ancle, elbow, and shoulder which need to be locked at the time of completely extending or in the middle of the extending or joints of robots. Some joints need to be locked at the time of bending or in the middle of the bending, and the joint mechanism 3 is also applicable to such cases. Further, the joint mechanism 3 is not limited for rehabilitation, but may be also applied to an assist device or the like to assist a joint, for example, when carrying heavies.

The joint member 19 of the present embodiment is provided with the leg frame part 15a and the thigh frame part 13a as the first turnable member and the second turnable member, and a clutch unit 23.

The leg frame part 15a is formed into a longitudinal plate and an upper end portion thereof in FIG. 1 (hereinafter, simply referred to as upper end portion) pivotally engages with the turning shaft 25. The turning shaft 25 is configured so that one end of a turning pin 25b is fit into an inner periphery of a turning sleeve 25a. The other end of the turning pin 25b is protruded in the axial direction. To an outer periphery of the turning sleeve 25a of the turning shaft 25, a fitting hole 27 formed in the leg frame part 15a is pivotally fitted.

The thigh frame part 13a is formed into a longitudinal plate similar to the leg frame part 15a, and a pair of connecting plates 31a, 31b are integrally provided to a lower end portion thereof in FIG. 1 (hereinafter, simply referred to as lower and portion). The pair of connecting plates 31a, 31b are fixed to and along the top and bottom of the thigh frame part 13a and are fixed to the turning shaft 25.

Accordingly, the thigh frame part 13a and the leg frame part 15a are configured to be pivotally connected to each other through the turning shaft 25.

To the pair of the connecting plates 31a, 31b of the thigh frame part 13a, a cylindrical lock member 33 is slidably externally fitted. The lock member 33 is movable between a lock position and an unlock position by the sliding, disables the thigh frame part 13a and the leg frame part 15a for turning at the lock position, and enables the thigh frame part 13a and the leg frame part 15a to turn at the unlock position.

The clutch unit 23 is detachably attached to the thigh frame part 13a and the leg frame part 15a to lock the turning between the thigh frame part 13a and the leg frame part 15a. It should be noted that the clutch unit 23 may be fixedly (undetachably) attached to the thigh frame part 13a and the leg frame part 15a.

The clutch unit 23 is provided with a first attachment member 35, the one-way clutch 37, a connecting part 39, a second attachment member 41, a fixing block 43 as a fixing member, and an attachment part 45.

The first attachment member 35 is a member engaging with the leg frame part 15a in the turning direction. The first attachment member 35 of the present embodiment has an arm shape provided along the upper end portion of the leg frame part 15a and has an engagement piece 35a.

The engagement piece 35a is fixed to the first attachment member 35 using a fastener 36 such as screw. The engagement piece 35a makes the first attachment member 35 engage with the leg frame part 15a in the turning direction.

The first attachment member 35 has a clutch supporting portion 35b positioned over the turning shaft 25. On the clutch supporting portion 35b, the one-way clutch 37 is fixed.

The one-way clutch 37 is connected to the leg frame part 15a and the thigh frame part 13a and is to lock the turning between the leg frame part 15a and the thigh frame part 13a to allow the turning in one direction (extending direction) only.

The one-way clutch 37 of the present embodiment can employ various types such as sprag type, cam type, and ratchet type and has engaging/disengaging members, not illustrated, such as sprags capable of engaging/disengaging between an inner part and an outer part.

The one-way clutch 37 has a cylindrical shape as a whole, and a fixing shaft 47 is inserted into a hole 37a formed on an inner periphery of the one-way clutch. The fixing shaft 47 has a cylindrical shape with an insertion hole 47a at an axial center portion, the other end of the turning pin 25b is inserted into the insertion hole 47a in the axial direction, and the fixing shaft is fixed to the clutch supporting portion 35a of the first attachment member 35 using a fastener 49 such as screw.

The one-way clutch 37 is, therefore, arranged concentrically with the turning shaft 25.

The fixing shaft 47 is provided with a flange portion 47b, and the one-way clutch 37 is supported between the flange portion 47b of the fixing shaft 47 and the clutch supporting portion 35b. With this supporting, the inner part of the one-way clutch 37 and the leg frame part 15a are configured to turn integrally.

On an outer periphery of the one-way clutch 37, the connecting part 39 is provided.

The connecting part 39 is to connect the thigh frame part 13a to the one-way clutch 37. In the connecting part 39 of the present embodiment, a connecting flange 39b is integrally provided on an outer periphery of a cylindrical portion 39a. It should be noted that the connecting part 39 should connect the thigh frame part 13a to the one-way clutch 37 and is not limited to the above configuration.

An inner periphery of the cylindrical portion 39a is fitted and connected to the outer periphery of the one-way clutch 37. With this, the connecting part 39 is configured to rotate integrally with the outer part of the one-way clutch 37.

It should be noted that the connecting part 39 may be integrally provided to the outer part of the one-way clutch 37. In this case, the cylindrical portion 39a may be omitted and only the connecting flange 39b may be integrally provided to the outer part of the one-way clutch 37.

A front end of the cylindrical portion 39a in the axial direction is protruded relatively to the connecting flange 39b.

The connecting flange 39b is provided with engagement holes 51a so as to pass therethrough in the axial direction, the engagement holes with which second engagement portions 71a of the connecting/disconnecting part 21 explained later engages. Further, the connecting flange 39 is provided with communicating holes 53a so as to pass therethrough in the axial direction, the communicating holes to be used in the embodiment 2.

The connecting part 39 supports a second attachment member 41. The second attachment member 41 engages with the thigh frame part 13a in the turning direction. The second attachment member 41 has a plate shape as a whole, and is provided integrally with a relatively rotatable portion 41a and an arm portion 41b.

The relatively rotatable portion 41a is relatively rotatably supported on the outer periphery of the one-way clutch 37. According to the present embodiment, the relatively rotatable portion is formed into a ring shape, and a fitting hole 55 formed on an inner periphery of the relatively rotatable portion is relatively rotatably fitted on the outer periphery of the cylindrical portion 39a of the connecting part 39. Accordingly, the relatively rotatable portion 41a is arranged to overlap the connecting flange 39b of the connecting part 39 in the axial direction, and is rotatable around the turning axis relatively to the connecting flange.

The relatively rotatable portion 41a is in contact with the connecting flange 39b on one side in the axial direction, and is supported with a stopper ring 57 on the other side in the axial direction to be axially positioned.

The relatively rotatable portion 41a is provided with a plurality of the communicating holes 53b in a circumferential direction, the communicating holes to be used in the embodiment 2. Each communicating hole 53b passes through the relatively rotatable portion 41a in the axial direction. These communicating holes 53b selectively communicate with the communicating holes 53a of the connecting part 39 in the axial direction according to a relative rotation state of the relatively rotatable portion 41a with respect to the connecting part 39.

The arm portion 41b of the second attachment member 41 is provided so as to protrude from the outer periphery of the relatively rotatable portion 41a in a radial direction. The arm portion 41b is provided along the lower end portion of the thigh frame part 13a, and has an engagement piece 41c engaging with the thigh frame part 13a. The engagement piece 41c makes the second attachment member 41 engage with the thigh frame part 13a on one side in the turning direction.

On the arm portion 41b, a protruding portion 59 is provided and protruded in the axial direction, the protruding portion serving as a displacement part with which a first engagement portion 69a of the connecting/disconnecting part 21 explained later engages. With this, the protruding portion 59 is arranged apart from the connecting part 39 in an intersecting direction intersecting the turning shaft 25 and is relatively turnable around the turning shaft 25.

Namely, the protruding portion 59 is provided to the thigh frame part 13a, and is configured to displace relatively to the connecting part 39 according to the turning between the leg frame part 15a and the thigh frame part 13a during the disconnecting of the one-way clutch 37 and the thigh frame part 13a.

The protruding portion 59 has an engagement recess 59a to receive the first engagement portion 69a, and engages with the first engagement portion 69a. It should be noted that the protruding portion 59 is not necessarily provided to the second attachment member 41, and may be directly provided to the thigh frame part 13a, for example.

To the engagement piece 41c of the arm portion 41b of the second attachment member 41, the fixing block 43 is attached as a fixing member, to detachably fix the clutch unit 23 to the thigh frame part 13a and the leg frame part 15a.

The fixing block 43 has slits 43a, 43b extending in different directions. The one slit 43a receives the thigh frame part 13a in the turning direction, to make the fixing block 43 engage with the top and the bottom of the thigh frame part 13a and with the other side thereof in the turning direction. The other slit 43b receives the engagement piece 41c of the second attachment member 41, to make the fixing block 43 engage with both sides of the engagement piece 41c in the turning direction. In this state, the fixing block 43 is fixed to the engagement piece 41c of the second attachment member 41 using a fastener 61 such as screw.

The fixing block 43, therefore, makes the second attachment member 41 detachably fix to the thigh frame part 13a. In this state, the protruding portion 59 of the clutch unit 23 is configured to turn integrally with the thigh frame part 13a.

Then, the connecting/disconnecting pat 21 connects and disconnects between the protruding portion 59 and the connecting part 39, whereby the joint mechanism 3 performs the locking and releases the locking relatively to the turning between the thigh orthosis 7 and the leg orthosis 9 of the long lower limb brace 1.

The attachment part 45 is to attach the connecting/disconnecting part 21 to the one-way clutch 37. The attachment part 45 of the present embodiment enables the connecting/disconnecting part 21 to be detachably connected to the one-way clutch 37. It should be noted that the connecting/disconnecting part 21 may be fixedly (undetachably) attached to the one-way clutch 37 or the clutch unit 23.

The attachment part 45 is fixed to the top of the connecting flange 39b of the connecting part 39 using a fastener 63 such as screw in the stacked state. The attachment part 45 of the present embodiment is formed into a ring shape, an inner periphery of which is fitted to the outer periphery of the axial front end of the cylindrical portion 39a of the connecting part 39.

The attachment part 45 is provided with engagement holes 51b in the axial direction, the engagement holes communicating with the engagement hole 51a of the connecting flange 39b. The engagement holes 51b as well as the engagement holes 51a of the connecting part 39 are engaged with the second engagement portions 71a of the connecting/disconnecting part 21. Further, the attachment part 45 is provided with communicating holes 53 in the axial direction, the communicating holes to be used in the embodiment 2.

On the bottom side of the attachment part 45, a retainer plate 65 is provided. The retainer plate 65 is formed into a ring shape, and is fitted to the outer periphery of the front end of the cylindrical portion 39a of the connecting part 39. The retainer plate 65 is accommodated in a recessed portion 45a formed on the bottom of the attachment part 45, is rotatable in a given range and is urged in a rotational direction using an urging member 67 such as spring.

The retainer plate 65 is provided with engagement holes 51c in the axial direction. The engagement holes 51c of the retainer plate 65 are arranged by the retainer plate 65 displaced and urged with the urging member 67 so as to be slightly displaced relatively to the engagement holes 51b of the attachment part 45 and the engagement holes 51a of the connecting part 39 in the rotational direction. Then, the engagement holes 51c of the retainer plate 65 axially communicate with the engagement holes 51a of the connecting part 39 and the engagement holes 51b of the attachment part 45 in a state that the displacement in the rotational direction is disappeared by displacing the retainer plate 65 against urging force of the urging member 67.

The retainer plate 65 of the present embodiment radially partly protrudes from the recessed portion 45a of the attachment part 45, and is configured to be displaced by manipulating the protruding portions.

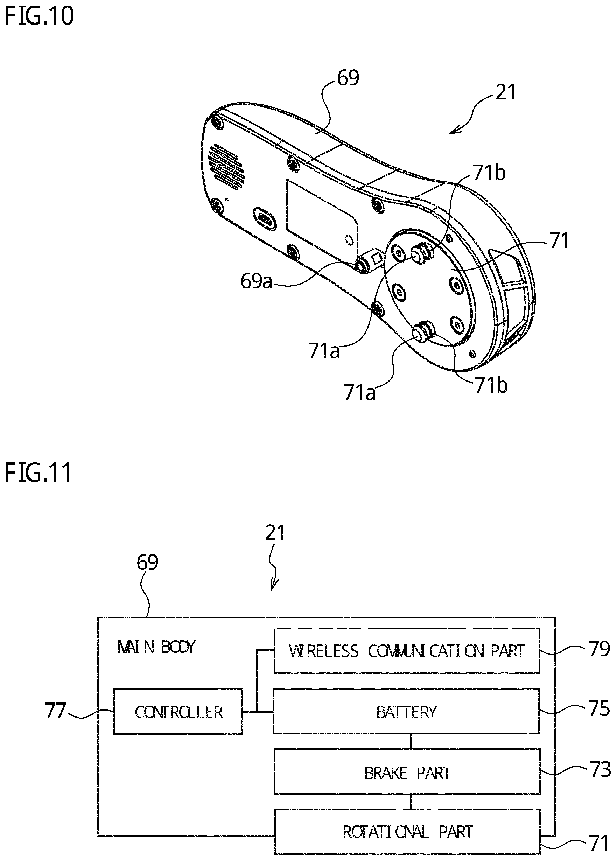

FIG. 10 is a perspective view of the connecting/disconnecting part used in the joint mechanism of FIG. 2 when viewed from the bottom side, and FIG. 11 is a block diagram illustrating the connecting/disconnecting part 21 of FIG. 10.

The connecting/disconnecting part 21 is to connect and disconnect a connecting state between the one-way clutch 37 and the thigh frame part 13a. According to this connecting and disconnecting, the turning between the thigh frame part 13a (thigh orthosis 7) and the leg frame part 15a (leg orthosis 9) is locked by connecting between the one-way clutch 37 and the thigh frame part 13a. With this locking, that turning is restricted in the bending direction and is allowed in the extending direction only. On the other hand, the turning between the thigh frame part 13a (thigh orthosis 7) and the leg frame part 15a (leg orthosis 9) is unlocked by disconnecting between the one-way clutch 37 and the thigh frame part 13a, to be allowed in both the extending direction and the bending direction.

In particular, the connecting/disconnecting part 21 of the present embodiment connects and disconnects between the connecting part 39 and the protruding portion 59 of the second attachment member 41 of the clutch unit 23, so as to connect the two to be undisplaceable to connect the thigh frame part 13a to the one-way clutch 37 and so as to disconnect the two to be displaceable to disconnect the thigh frame part 13a from the one-way clutch 37.

The connecting/disconnecting part 21 may be either manual or electric, and is electric in the present embodiment. The connecting/disconnecting part 21 is provided with a main body 69, a rotational part 71, and a brake part 73.

In the main body 69, a battery 75 to perform power supply to the brake part 73, a controller 77 to control the power supply, and a wireless communication part 79 and the like are accommodated.

It should be noted that the controller 77 is an arithmetic unit such as processor. The wireless communication part 79 is a unit capable of at least receiving signals, to allows instructions for the power supply and the release of the power supply to be wirelessly input into the controller 77.

The instructions for the power supply and the release of the power supply may be performed by a wireless operating device such as remote controller to be manipulated by an aided person or a helper thereof. Instead of the wireless communication part 79 and the controller 77, a switch may be provided to the connecting/disconnecting part 21 to conduct the power supply and the release of the power supply.

The main body 69 has the first engagement portion 69a to engage with the second attachment member 41. The first engagement portion 69a of the present embodiment is a pin and protrudes from the main body 69 to engage with the engagement recess 59a of the protruding portion 59. It should be noted that the first engagement portion 69a may be an engagement pawl or the like to hold the second attachment member 41. In this case, the second attachment member 41 as it is may be the displacement part.

The rotational part 71 is supported rotatably around the turning shaft 25 with the main body 69 and engages with the connecting part 39. The rotational part 71 of the present embodiment is attached to and on the attachment part 45 of the clutch unit 23 using the second engagement portions 71a and engages with the connecting part 39. It should be noted that the second engagement portions 71a may be engagement pawls or the like to hold the connecting flange 39b of the connecting part 39.

The second engagement portions 71a of the present embodiment are formed into a pin shape and axially protrude from the rotational part 71. The second engagement portions 71a pass through the engagement holes 51b of the attachment part 45 and the engagement holes 51c of the retainer plate 65 and front ends thereof reach the engagement holes 51a of the connecting flange 39b of the connecting part 39.

The second engagement portion 71a is provided with a narrow portion 71b at an axial position corresponding to the retainer plate 65, and the retainer plate 65 engages with the narrow portion 71b due to the urging force of the urging member 67 so as to be retained. With this, the connecting/disconnecting part 21 is detachably attached to the clutch unit 23 (one-way clutch 37).

The brake part 73 is arranged between the main body 69 and the rotational part 71, to apply or release load torque with respect to rotation of the rotational part 71. Accordingly, the brake part 73 brakes the rotational part 71 relatively to the main body 69 and release the braking, to connect and disconnect the protruding portion 59 of the clutch unit 23 with respect to the connecting part 39.

The brake part 73 may employ an electromagnetic clutch using ERG (Electrorheological gel) proposed by the present applicant as JP patent application No. 2015-42789, or the other electromagnetic clutch using frictional plates or magnetic fluid or the like, or various electromagnetic clutches or brakes.

### [Operation of long lower limb brace]

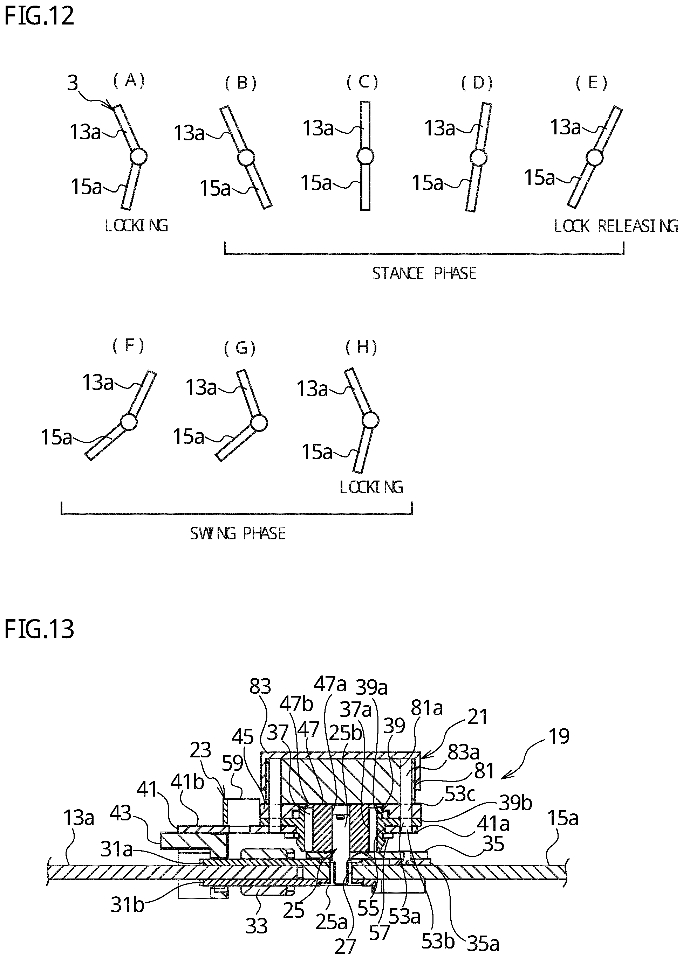

Hereinafter, the operation of the long lower limb brace 1 will be explained referring to also FIG. 12. It should be noted that the long lower limb brace 1 is for a right lower limb and a case that the long lower limb brace 1 is attached to a right lower limb will be explained.

FIG. 12 is a schematic view illustrating a relation between motion and locking of the leg frame part 15a and the thigh frame part 13a of the joint mechanism 3.

The long lower limb brace 1 of the present embodiment, when an aided person walks with long lower limb brace attached to a lower limb of the aided person, performs locking and release the locking, thereby to assist around a knee during the walking of the aided person and make the gait of the aided person natural.

In particular, when the aided person walks, the tuning of the thigh frame part 13a and the leg frame part 15a is locked in the process (FIG. 12(A)) of the extending between the thigh frame parts 13a, 13b (thigh orthosis 7) and the leg frame parts 15a, 15b (leg orthosis 9) until a heel of the right foot is put on the floor.

The locking is performed by applying the load torque between the rotational part 71 and the main body 69 with respect to the rotation of the rotational part 71 using the brake part 73 of the connecting/disconnecting part 21. With this, the thigh frame part 13a of the joint mechanism 3 is connected to the one-way clutch 37 through the protruding portion 59 of the clutch unit 23, the connecting/disconnecting part 21, and the connecting part 39 of the clutch unit 23.

The one-way clutch 37 is, therefore, in a state of being connected to the thigh frame part 13a and the leg frame part 15a, locks the relatively turning therebetween and allow the relatively turning in the extending direction only.

As a result, even if the locking is performed in the process of the extending between the thigh frame parts 13a, 13b (thigh orthosis 7) and the leg frame parts 15a, 15b (leg orthosis 9), operation in the extending direction is kept on performing between the thigh frame parts 13a, 13b (thigh orthosis 7) and the leg frame parts 15a, 15b (leg orthosis 9) according to the walking state of the aided person and it is possible to perform the locking in the state of completely extending or in the middle of the extending according to the gait of the aided person. It should be noted that, since the knee cannot be completely extended according to the aided person, there may be a need to perform the locking in the middle of the extending. FIGs. 12(B)-(H), however, illustrate the case of the completely extending.

Then, the heel of the right foot is put on a floor in the state that the thigh frame parts 13a, 13b (thigh orthosis 7) and the leg frame parts 15a, 15b (leg orthosis 9) are completely extended, whereby the aided person is prevented from a knee bending of the right lower limb and transitions to a stance phase (state of applying the body weight) with the natural gait (FIGs. 12(B)-(E)).

After transitioning to the stance phase, the locking to the turning of the thigh frame parts 13a, 13b (thigh orthosis 7) and the leg frame parts 15a, 15b (leg orthosis 9) is released when leaving the heel of the right foot from the floor, whereby a toe is left from the floor while the knee of the right lower limb of the aided person bends naturally, to transition to a swing phase (FIGs. 12(F)-(H)).

After transitions to the swinging phase, the tuning of the thigh frame part 13a and the leg frame part 15a is locked again using the joint mechanism 3 in the process of the extending between the thigh frame parts 13a, 13b (thigh orthosis 7) and the leg frame parts 15a, 15b (leg orthosis 9) until the heel of the right foot is put on the floor.

In this way, the locking and the release of the locking with respect to the tuning of the thigh frame parts 13a, 13b (thigh orthosis 7) and the leg frame parts 15a, 15b (leg orthosis 9) are repeated, thereby to make the aided person walk with the natural gait while preventing the knee bending.

It should be noted that the long lower limb brace 1 of the present embodiment may perform locking in a sitting state in which a thigh and a leg of an aided person are bent to assist extension of the thigh and the leg at the time of standing as well as walking.

### [Effect of embodiment 1]

As mentioned above, the joint mechanism 3 used in the long lower limb brace 1 of the present embodiment is provided with the leg frame part 15a and the thigh frame part 13a pivotally connected to each other using the turning shaft 25, the one-way clutch 37 connected to the leg frame part 15a and the thigh frame part 13a so as to lock the turning between the leg frame part 15a and the thigh frame part 13a and allow the turning in one direction only, and the connecting/disconnecting part 21 to connect and disconnect the connecting state between the one-way clutch 37 and the thigh frame part 13a, wherein the connecting/disconnecting part 21 disconnects between the one-way clutch 37 and the leg frame part 15a to release the locking of the turning between the leg frame part 15a and the thigh frame part 13a to allow the leg frame part 15a and the thigh frame part 13a to turn in both directions.

The joint mechanism 3, therefore, connects between the one-way clutch 37 and the thigh frame part 13a using the connecting/disconnecting part 21 to lock the turning between the leg frame part 15a and the thigh frame part 13a so as to allow the turning in the extending direction only.

As a result, if the locking is performed in the process of the extending between the leg frame part 15a and the thigh frame part 13a, operation in the extending direction is kept on performing between the leg frame part 15a and the thigh frame part 13a and it is possible to perform the locking in the state of completely extending or in the middle of the extending.

Accordingly, the joint mechanism 3 is lockable in the state of completely extending or in the middle of the extending without complicated control with the simple structure while suppressing complication and weight increase.
Further, since the joint mechanism 3 does not need the complicated control, a helper or an aided person operates the locking and the unlocking.

The joint mechanism 3 of the present embodiment is provided with the connecting part 39 provided to the one-way clutch 37 to connect the thigh frame part 13a, and the protruding portion 59 provided to the thigh frame part 13a and configured to displace relatively to the connecting part 39 according to the turning between the leg frame part 15a and the thigh frame part 13a during the disconnecting between the one-way clutch 37 and the thigh frame part 13a, wherein the connecting/disconnecting part 21 connects between the connecting part 39 and the protruding portion 59 to disable for the displacing therebetween, thereby to connect the thigh frame part 13a to the one-way clutch 37.

The present embodiment, therefore, surely performs the connecting and the disconnecting between the thigh frame part 13a and the one-way clutch 37.

In particular, according to the present embodiment, the connecting part 39 and the protruding portion 59 are arranged to be apart from each other in the intersecting direction intersecting the turning shaft 25 and are relatively rotatable around the turning shaft 25, and the connecting/disconnecting part 21 is provided with the main body 69 having the first engagement portion 69a engaging with the protruding portion 59, the rotational part 71 supported rotatably around the turning shaft 25 of the main body 69 and having the second engagement portions 71a engaging with the connecting part 39, and the brake part 73 braking the rotation of the rotational part 71 and releasing the braking to connect and disconnect the thigh frame part 13a and the one-way clutch 37.

The present embodiment, therefore, performs the connecting and the disconnecting of the thigh frame part 13a to the one-way clutch 37 with the braking of the rotational part 71 using the brake part 73 and the releasing of the braking, and accordingly reduces the size of the brake part 73 and reduces the size of the connecting/disconnecting part 21 as a whole.

Further, since the connecting/disconnecting part 21 of the present embodiment accommodates the brake part 73, the battery 75 and the controller 77 to control the brake part 73 and the like in the main body 69, it further reduces the size as whole.

Further, the connecting/disconnecting part 21 is detachably attached to the one-way clutch 37, and charging or the like is easily performed thereto.

The long lower limb brace 1 using the joint mechanism 3 is provided with the thigh orthosis 7 provided to the thigh frame part 13a of the joint mechanism 3 to be attached to a thigh of an aided person, and the leg orthosis 9 provided to the leg frame part 15a of the joint mechanism 3 to be attached to a leg of the aided person, wherein the joint mechanism 3 locks the turning between the leg frame part 15a and the thigh frame part 13a to allow the thigh orthosis 7 and the leg orthosis 9 to turn in the extending direction only and releases the locking of the turning to allow the thigh orthosis 7 and the leg orthosis 9 to turn in both directions of the extending direction and the bending direction.

The long lower limb brace 1 of the present embodiment, therefore, locks the turning in the process of the extending between the thigh orthosis 7 and the leg orthosis 9, thereby to keep the thigh orthosis 7 and the leg orthosis 9 operating in the extending direction and it is possible to perform the locking in the state of completely extending or in the middle of the extending.

Then, the heel of the aided person is put on the floor in the locked state between the thigh orthosis 7 and the leg orthosis 9, whereby the aided person is prevented from a knee bending and naturally transitions to the stance phase.

Further, the locking of the turning of the thigh orthosis 7 and the leg orthosis 9 are released when leaving the heel from the floor in the stance phase, whereby the heel is left from the floor while the aided person naturally bends the knee to transition to the swing phase.

In this way, the long lower limb brace 1 of the present embodiment repeats the locking and the releasing of the locking with respect to the tuning of the thigh orthosis 7 and the leg orthosis 9, thereby to make the aided person walk with the natural gait while preventing the knee bending of the aided person with the simple control.

Further, the joint member 19 used in the joint mechanism 3 is provided with the leg frame part 15a and the thigh frame part 13a pivotally connected to each other using the turning shaft 25, the one-way clutch 37 connected to the leg frame part 15a and the thigh frame part 13a to lock the turning between the leg frame part 15a and the thigh frame part 13a and allow the turning in one direction only, and the attachment part 45 for attaching the connecting/disconnecting part 21 to connect and disconnect the connecting state between the one-way clutch 37 and the thigh frame part 13a.

The joint member 19, therefore, easily forms the joint mechanism 3 by the attachment of the connecting/disconnecting part 21.

The clutch unit 23 used in the joint mechanism 3 is provided with the first attachment member 35 and the second attachment member 41 engaging with the leg frame part 15a and the thigh frame part 13a in the turning direction, the fixing block 43 detachably fixing the second attachment member 41 to the leg frame part 15a, the one-way clutch 37 supported with the leg frame part 15a to lock the turning between the leg frame part 15a and the thigh frame part 13a to allow the turning in one direction only, the relatively rotatable part 41a provided to the second attachment member 41a and relatively rotatably supported with the outer periphery of the one-way clutch 37, and the attachment part 45 for attaching the connecting/disconnecting part 21 to connect and disconnect the connecting state between the one-way clutch 37 and the thigh frame part 13a.

The clutch unit 23, therefor, easily forms the joint member 19 by detachably attaching the one-way clutch 37 to the leg frame part 15a and the thigh frame part 13a. Accordingly, the leg frame part 15a and the thigh frame part 13a are generalized to realize cost down. Further, the clutch unit 23 is capable of retrofitting the one-way clutch between the leg frame part 15a and the thigh frame part 13a to form the joint member 19.

### EMBODIMENT 2

FIG. 13 is a sectional view illustrating a joint mechanism according to the embodiment 2 of the present invention. In addition, the basic structure of the embodiment 2 is common with the embodiment 1, and therefore, components of the embodiment 2 corresponding to the embodiment 1 are represented with the same numerals to eliminate repetition in description.

The present embodiment is configured to manually perform locking and unlocking of a connecting/disconnecting part 21 of a joint mechanism 3. The others are the same as of the embodiment 1.

The connecting/disconnecting part 21 of the present embodiment is to connect and disconnect between a connecting flange 39b of a connecting part 39 and a relatively rotatable portion 41a as a displacement part which is arranged so as to overlap the connecting flange in an axial direction. The connecting/disconnecting part 21 is provided with a fixed part 81, and a movable part 83.

The fixed part 81 is a part attached to an attachment part 45 of a joint member 19. The fixed part 81 is formed into a round column shape, and is provided with second engagement portions 71a (not illustrated) similar to the rotational part 71 of the embodiment 1. Further, the fixed part 81 is provided with through holes 81a passing through the fixed part in the axial direction.

The movable part 83 is supported to the fixed part 81 so as to be slidable within a given range in the axial direction. To the movable part 83, pins 83a passing through the through holes 81a of the fixed part 81 are supported. The pins 83a are axially movable according to the slide of the movable part 83.

With this, the pins 83a are axially movably arranged in communicating holes 53c of the attachment part 45, communicating holes 53a of the connecting part 39, and communicating holes 53b of the relatively rotatable part 41a of the second attachment member 41, and conduct the connecting and the disconnecting between the one-way clutch 37 and a thigh frame part 13a according to the axial movement.

Namely, the pins 83a are inserted into the communicating holes 53c of the attachment part 45, the communicating holes 53a of the connecting part 39, and the communicating holes 53b of the second attachment member 41 to connect between the one-way clutch 37 and the thigh frame part 13a. On the other hand, the pins 83a are pulled off from the communicating holes 53b of the second attachment member 41, with the axial movement to disconnect between the one-way clutch 37 and the thigh frame part 13a.

The present embodiment, therefore, manually slides the movable part 83 relatively to the fixed part 81 of the connecting/disconnecting part 21, thereby to repeat the locking and the releasing of the locking with respect to the turning between the thigh frame part 13a (thigh orthosis 7) and the leg frame part 15a (leg orthosis 9), and it is possible to make an aided person walk with the natural gait while preventing the knee bending.

It should be noted that the movable part 83 may be configured to be electrically slid. Further, the connecting/disconnecting part 21 may be configured to directly move the pins 83a in the axial direction while omitting the movable part 83. Furthermore, in the connecting/disconnecting part 21, the fixed part 81 may be omitted. In a case that both the movable part 83 and the fixed part 81 are omitted, the pins 83a are configured to be supported in the communicating holes 53a, 53b, 53c.

In addition, the embodiment 2 provides the same effect as the embodiment 1.

### EMBODIMENT 3

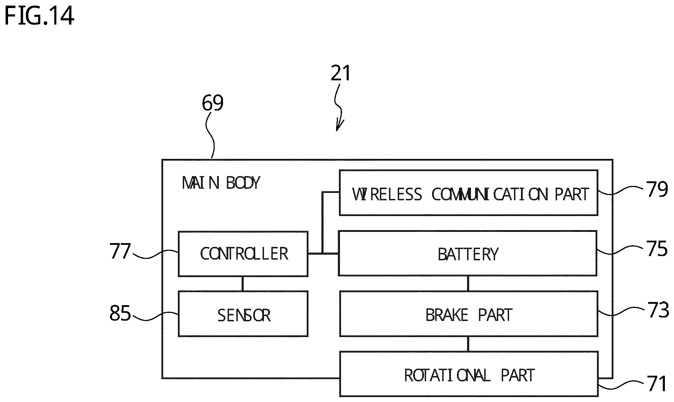

FIG. 14 is a block diagram illustrating a connecting/disconnecting part used in a joint mechanism according to the embodiment 3 of the present embodiment. In addition, the basic structure of the embodiment 3 is common with the embodiment 1, and therefore, components of the embodiment 3 corresponding to the embodiment 1 are represented with the same numerals to eliminate repetition in description.

The present embodiment automatically locks and releases the locking with respect to turning of a thigh frame part 13a (thigh orthosis 7) and a leg frame part 15a (leg orthosis 9). For this, a connecting/disconnecting part 21 is provided with a sensor 85.

The sensor 85 detects a front/rear position of a thigh of an aided person, and is configured by, for example, a gyro sensor, a tilt sensor or the like. It should be noted that the sensor 85 may be provided to the thigh frame part 13a. Further, the sensor 85 may be one attached to the thigh of the aided person, not to the joint mechanism 3. In this case, the front/rear information of the thigh is sent from the sensor 85 to the connecting/disconnecting part 21 side by wired or wireless communication.

The joint mechanism 3, similar to the embodiment 1, is configured to electrically switch the locking and the unlocking of the turning of the thigh frame part 13a (thigh orthosis 7) ad the leg frame part 15a (leg orthosis 9).

Then, the joint mechanism 3, based on the detection at the sensor 85, conducts the locking by that the controller 77 generates load torque on the brake part 73 of the connecting/disconnecting part 21 when the thigh of the aided person is positioned ahead of a center of gravity of the body of the aided person, and conducts the unlocking by that the load torque is removed when the thigh of the aided person is positioned behind the center of gravity of the center of the aided person.

With the locking and the unlocking, it automatically assists around the knee of the aided person at the time of the waking with the simple control and configuration, to make the gait natural.

In addition, the embodiment 3 provides the same effect as the embodiment 1.

### DESCRIPTION OF REFERENCE NUMERALS

- 1: Long lower limb brace (knee joint assist device)
- 3: Joint mechanism
- 7: Thigh orthosis
- 9: Leg orthosis
- 13a: Thigh frame part (second turnable member)
- 15a: Leg frame part (first turnable member)
- 19: Joint member
- 21: Connecting/disconnecting part
- 23: Clutch unit
- 25: Turning shaft
- 35: First attachment member
- 37: One-way clutch
- 39: Connecting part
- 41: Second attachment member
- 43: Fixing block
- 45: Attachment part
- 53a, 53b, 53c: Communicating hole
- 59: Displacement part
- 69: Main body (connecting/disconnecting part)
- 69a: First engagement portion
- 71: Rotational part
- 71a: Second engagement portion
- 73: Brake part
- 83a: Pin
- 85: Sensor

## Claims

1. Ajoint mechanism comprising:
a first turnable member and a second turnable member pivotally connected to each other;
a one-way clutch connected to the first turnable member and the second turnable member so as to lock turning between the first turnable member and the second turnable member and allow the turning in one direction only; and
a connecting/disconnecting part to connect and disconnect a connecting state between the one-way clutch and the second turnable member, wherein
the connecting/disconnecting part disconnects between the one-way clutch and the second turnable member to release the locking of the turning to allow the first turnable member and the second turnable member to turn in both directions.

2. The joint mechanism according to claim 1, further comprising:
a connecting part provided to the one-way clutch to connect the second turnable member; and
a displacement part provided to the second turnable member and configured to displace relatively to the connecting part according to the turning between the first turnable member and the second turnable member during the disconnecting of the one-way clutch and the second turnable member, wherein
the connecting/disconnecting part connects between the connecting part and the displacement part to disable for the displacing, thereby to connect the second turnable member to the one-way clutch.

3. The joint mechanism according to claim 2, wherein
the connecting part and the displacement part are arranged to overlap each other in a turning axis direction, are relatively turnable around a turning axis, and have communicating holes communicating in the turning axis direction, and
the connecting/disconnecting part has a pin arranged in the communicating holes so as to be movable in an axis direction, and performs the connecting and the disconnecting according to the moving of the pin in the axis direction.

4. The joint mechanism according to claim 2, wherein
the connecting part and the displacement part are arranged apart from each other in an intersecting direction intersecting a turning axis and are relatively rotatable around the turning axis,
the connecting/disconnecting part comprises:
a body portion having a first engagement portion engaging with the displacement part;
a rotational part supported with the body portion rotatably around the turning axis and having a second engagement portion engaging with the connecting part; and
a brake part configured to brake rotation of the rotational part and release the braking to conduct the connecting and the disconnecting.

5. The joint mechanism according to any one of claims 1-4, wherein
the connecting/disconnecting part is detachably attached to the one-way clutch.

6. A knee joint assist device using the joint mechanism according to any one of claims 1-5, comprising:
a thigh orthosis provided to one of the first turnable member and the second turnable member of the joint mechanism to be attached to a thigh of an aided person; and
a leg orthosis provided to the other of the first turnable member and the second turnable member of the joint mechanism to be attached to a leg of the aided person, wherein
the joint mechanism locks the turning between the first turnable member and the second turnable member to allow the thigh orthosis and the leg orthosis to turn in an extending direction only and releases the locking to allow the thigh orthosis and the leg orthosis to turn in both directions of the extending direction and a bending direction.

7. The knee joint assist device according to claim 6, further comprising:
a sensor that detects a front/rear position of the thigh of the aided person, wherein
the joint mechanism is configured to electrically switch the locking of the turning and the release the locking,
the locking of the turning is performed when the thigh of the aided person is positioned ahead of a center of gravity of the aided person and the locking of the turning is released when the thigh of the aided person is positioned behind the center of gravity of the aided person.

8. Ajoint member comprising:
a first turnable member and a second turnable member pivotally connected to each other;
a one-way clutch connected to the first turnable member and the second turnable member to lock turning between the first turnable member and the second turnable member and allow the turning in one direction only; and
an attachment part for attaching a connecting/disconnecting part to connect and disconnect a connecting state between the one-way clutch and the second turnable member.

9. A clutch unit detachably attached to a first turnable member and a second turnable member to lock turning between the first turnable member and the second turnable member and allow the turning in one direction only, comprising:
a first attachment member and a second attachment member engaging with the first turnable member and the second turnable member in a turning direction to be attached to the first turnable member and the second turnable member;
a fixing member detachably fixing the first attachment member or the second attachment member to the first turnable member or the second turnable member;
a one-way clutch supported with one of the first attachment member and the second attachment member to lock turning between the first turnable member and the second turnable member and allow the turning in one direction only;
a relatively rotatable part provided to the other of the first attachment member and the second attachment member and relatively rotatably supported with an outer periphery of the one-way clutch; and
an attachment part for attaching a connecting/disconnecting part to connect and disconnect a connecting state between the one-way clutch and the second turnable member.
